(19) Europäisches Patentamt
European Patent Office
Office européen des brevets

(11) EP 2 776 006 B1

(12) EUROPEAN PATENT SPECIFICATION

(45) Date of publication and mention of the grant of the patent:
**23.09.2015 Bulletin 2015/39**

(21) Application number: **12781320.2**

(22) Date of filing: **06.11.2012**

(51) Int Cl.:
*A61K 8/81* (2006.01) *A61K 8/365* (2006.01)
*A61Q 15/00* (2006.01) *A61K 8/92* (2006.01)

(86) International application number:
**PCT/EP2012/071929**

(87) International publication number:
**WO 2013/068345 (16.05.2013 Gazette 2013/20)**

# (54) ORGANOGEL STRUCTURED WITH 12-HSA AND A SELECTED COPOLYMER

ORGANOGEL MIT 12-HSA UND EINEM AUSGEWÄHLTEN COPOLYMER

ORGANOGEL À STRUCTURE 12-HSA ET UN COPOLYMÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.11.2011 US 201161558780 P**

(43) Date of publication of application:
**17.09.2014 Bulletin 2014/38**

(73) Proprietors:
- **Unilever PLC**
**London, Greater London EC4Y 0DY (GB)**
Designated Contracting States:
**CY GB IE MT**
- **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**

(72) Inventors:
- **LITVIN, Tamara**
**Trumbull, CT 06611 (US)**
- **LIPS, Alexander**
**Neston**
**Cheshire CH64 6SQ (GB)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(56) References cited:
**WO-A1-95/31961**
**WO-A1-2008/037697**
**WO-A1-2010/119035**
**WO-A2-02/43685**
**WO-A2-2010/119034**
**WO-A2-2012/080272**
**DE-A1- 10 317 751**

- **ANONYMOUS: "Sales Specification Ganex V-220", INTERNET CITATION, 24 May 2006 (2006-05-24), page 1, XP002593625, Retrieved from the Internet: URL:http:// online1.ispcorp.com/Specs/11_GA NEX%20V-220.pdf [retrieved on 2010-07-22]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 776 006 B1

## Description

FIELD OF THE INVENTION

The subject invention relates to compositions, in particular, to cosmetic and personal care compositions in the form of organogels and organogel-containing compositions including, but not limited to, antiperspirant compositions.

Background of the Invention

Organogels are thermally reversible solid structures having a liquid organic phase entrapped in a three-dimensional, cross-linked network structure. Organogels can vary in terms of their rigidity depending upon the selection and amount of the network-forming structurant.

The structure of an organogel is typically formed by organic gelator molecules precipitating out of the organic phase and forming clusters of fibers or filaments that grow from one or several nucleation centers. On a microscopic scale, these clusters give rise to the formation of crystalline regions within which oils and other fluid components that form the liquid organic phase may become trapped or entrained. The ability of an organogel to retain fluid within network clusters is determined by factors that can include size, density, extent of cross-linking, and uniformity of crystalline regions, as well as on the size of crystalline regions relative to non-crystalline regions of the gel. That is to say, a more uniform or homogenous gel structure may result when fluid regions within crystalline clusters more closely "match" the size of inter-cluster boundaries. A more homogenous gel structure can give rise to improved gel stability and fracture resistance as well as to improved fluid retention.

In organogels, formation of a network structure is normally reversible in that, at temperature at which the gelator becomes soluble in the organic phase, the network structure of the gelator collapses, with the structure re-forming when brought to temperature at which the solubility of the gelator in the organic phase is exceeded.

Many applications for organogels are as anhydrous compositions, however, compositions that include water or other hydrophilic components are also of interest, as are compositions in which the organogel itself is dispersed in a separate carrier phase, i.e., organogel-containing compositions.

A gelator of particular interest for many applications is 12-hydroxystearic acid ("12-HSA"). 12-HSA and salts thereof are known as small molecule gelling agents. See, for example, Tsau et al., "Thermoreversible Organogels of 12-Hydroxystearic Acid", American Chemical Society Polymer Preprints, 1994, vol. 35, pp 737-738 and Tamura et al. "Effect of Alkali Metal Ions on Gel Formation in the 12-Hydroxystearic Acid/Soybean Oil System", JAOCX, vo. 68, No. 8 (August, 1991).

U.S. Pat. No. 5,429,816 discloses antiperspirant sticks that contain an antiperspirant active, a suitable liquid carrier and up to about 15% by weight of a low residue gellant such as 12-hydroxystearic acid. U.S. Pat. No. 6,352,688 discloses an anhydrous antiperspirant stick formulation that comprises (a) from about 0.5% to about 60% by weight of particulate antiperspirant active; (b) from about 3% to about 50% by weight of a solid suspending agent containing a first suspending agent and a second suspending agent; and (c) from about 10% to about 80% by weight of a carrier liquid, preferably a volatile and/or non volatile silicone, wherein: the first and second suspending agents are solids at human skin temperature; the first and second suspending agents together have a melt point of less than 120°C, the second suspending agent having a lower melt point than the first suspending agent; and the second suspending agent is liquified during formulation of the antiperspirant composition and then used in liquid form to solubilize within the composition the first suspending agent at a temperature less than 120°C. 12-hydroxystearic acid is among the materials described as being suitable for use as the first suspending agent.

U.S. Pat. Publn. No. 2009/0317341 discloses cosmetic compositions that comprise from about 0.001 to about 15% by weight of skin lightening additive, which additive may be 12-hydroxystearic acid.

WO95/31961 discloses antiperspirant gel stick compositions comprising: (a) an antiperspirant active; (b) a gelling agent comprising: (i) an optically enriched primary gellant selected from the group consisting of 12-hydroxystearic acid, esters of 12-hydroxystearic acid, amides of 12-hydroxystearic acid, and mixtures thereof, and (ii) a secondary gellant selected from the group consisting of n-acyl amino acid amide derivatives; (c) a liquid base material having a solubility parameter of 9 or below; wherein the gel stick has a hardness of 75 grams of force or more.

WO02/43685 discloses a cosmetic and/or pharmaceutical composition containing: (a) an oil component; (b) a viscosity modifier selected from the group consisting of a $C_{6-22}$ hydroxyfatty acid, a salt of a $C_{6-22}$ hydroxyfatty acid, and mixtures thereof; and (c) water.

DE 103 17 751 discloses an anhydrous antiperspirant composition in semisolid to solid form comprises an antiperspirant, a volatile carrier, a non-volatile emollient and a thickener system comprising hydroxyoctacosanyl 12-hydroxystearate and an inorganic and/or polymeric thickener.

WO2008/037697 discloses nanospheres of organogel comprising a low-molecular-weight organogelling agent capable of gelling a cosmetic or pharmaceutical active ingredient which is lipophilic or amphiphilic, non-volatile and liquid

at ambient temperature. The low-molecular-weight organogelling agent can be 12-hydroxystearic acid.

WO2010/119035 discloses silicone oils which are structured with a copolymer comprising a large percentage of alkane groups. Using high percentage pendant alkane helps provide silicone with beneficial gel properties and viscosity.

WO2010/119034 discloses petrolatum based composition containing silicone oils which are structured with specific blend and ratio of fatty acids.

Even when used at relatively low levels, 12-HSA tends to give rise to relatively rigid gels that have limited capacity to retain organic fluids. WO2008/037697 discloses a process said to produce nanospheres of organogels (including organogels of 12-HSA) in which agents for dispersing and/or stabilizing the gel structure are incorporated in whole or part on the surface of the nanospheres.

One aspect of this invention is to improve the rheological properties of a 12-HSA-containing organogel, more particularly to provide rheological properties that give rise to softer more spreadable compositions. Yet another aspect of this invention is to provide 12-HSA-containing organogels capable of retaining higher levels of oil by preventing gel syneresis. In least one embodiment, another aspect of this invention is to provide 12-HSA-containing organogels in which desirable oil retention and rheological properties are achieved in gels that are substantially free of higher HLB surfactants, in particular anionic higher HLB surfactants, many of which surfactants are potentially harsher on skin than the more lipopohilic lower HLB surfactants.

BRIEF DESCRIPTION OF THE INVENTION

It has now been found that one or more aspects of this invention can be achieved in organogels that include a 12-HSA gelator and, as a crystal habit modifier, a selected copolymer having pendant alkyl groups In one embodiment of this invention there is provided an organogel comprising:

(a) 4 to 20% by weight of 12-hydroxystearic acid gelator;
(b) 45 to 95% by weight cosmetically acceptable oil;
(c) 0.05 to 3% by weight of a vinyl amide copolymer that carries at least 70% by weight of the copolymer of pendant alkyl groups having chain length of $C_{12}$-$C_{24}$, the copolymer preferably containing from 1 to less than 30% by weight of backbone monomer capable of forming a polymer backbone bearing such pendant alkane groups, for example, cyclic vinyl amide monomer and/or other monomers such as are described below.

In one or more embodiments of particular interest, the organogel is substantially free of surfactant having an HLB value greater than 6 and, in the case of anhydrous compositions, is preferably substantially free of surfactant having an HLB value greater than 4.

In another embodiment of this invention there is provided an organogel comprising:

(a) 4 to 20% by weight of 12-hydroxystearic acid gelator;
(b) 45 to 95% by weight cosmetically acceptable oil; and
(c) 0.05 to 3% by weight of a vinyl amide copolymer that carries at least 70% by weight of the copolymer of pendant alkyl groups having chain length of $C_{12}$-$C_{24}$, the copolymer preferably containing from 1 to less that 30% by weight of backbone monomer capable of forming a polymer backbone bearing such pendant alkyl groups;

wherein the organogel is substantially free of surfactant having an HLB value greater than that of the 12-hydroxystearic acid gelator of highest HLB value present in the organogel.

In another embodiment, the organogel is formulated as an antiperspirant composition that further comprises up to 30% by weight of antiperspirant active.

In other embodiments, the subject invention further comprises one or more skin care actives. In yet another embodiment of interest there is provided a cosmetic composition that includes the subject organogel as a component thereof, i.e., an organogel-containing composition.

These and other aspects, features and advantages will become apparent to those of ordinary skill in the art from a reading of the following detailed description and the appended claims. For the avoidance of doubt, any feature of one aspect of the present invention may be utilized in any other aspect of the invention. It is noted that the examples given in the description below are intended to clarify the invention and are not intended to limit the invention to those examples per se. Other than in the experimental examples, or where otherwise indicated, all numbers expressing quantities of ingredients or reaction conditions used herein are to be understood as modified in all instances by the term "about". Similarly, all percentages are weight/weight percentages of the total composition being referenced unless otherwise indicated. Numerical ranges expressed in the format "from x to y" are understood to include x and y. When for a specific feature multiple preferred ranges are described in the format "from x to y", it is understood that all ranges combining the different endpoints are also contemplated. Where the term "comprising" is used in the specification or

claims, it is not intended to exclude any terms, steps or features not specifically recited. All temperatures are in degrees Celsius (°C) unless specified otherwise. All measurements are in SI units unless specified otherwise. All documents cited are, in relevant part, incorporated herein by reference.

BRIEF DESCRIPTION OF THE FIGURES

Figure 1 is plot (G' and G" as a function of % strain at constant frequency) that compares the effect on rheology of the addition of Ganex® V-216 copolymer to a composition that includes 12-hydroxystearic acid and soybean oil.
Figure 2 is a plot (G' and G" as a function of frequency) that compares the effect on rheology of the addition of Ganex® V-216 copolymer to a composition that includes 12-hydroxystearic acid and soybean oil.
Figure 3 is a plot (G' and G" as a function of frequency) that compares the effect on rheology of the addition of Ganex® V-216 copolymer at different levels to a composition that includes 12-hydroxystearic acid and soybean oil.
Figure 4 is a photomicrographic comparison of the effect on microstructure of the addition of Ganex® V-220 copolymer to a composition that includes 12-hydroxystearic acid and soybean oil.

DETAILED DESCRIPTION OF THE INVENTION

It has been found that the crystal habit of organogels containing 12-hydroxystearic acid gelators can be modified by incorporating therein selected copolymers, producing an organogel having crystalline regions or domains ranging in size from 0.1 micron (or less) to less than 500 microns, more particularly less than 200 microns. In one or more embodiments, the organogel has a domain size on the order of from 1 to 100 microns and, more particularly, on the order of from 10 to 60 microns. Microscopy and x-ray diffraction are among the techniques from which domain size of gel structures are commonly derived.

The modified organogels have a relatively uniform structure in which the crystalline clusters more closely "match" the size of the inter-cluster boundaries. The modified organogels of the subject invention provide improved gel stability (e.g., improved oil retention) and fracture resistance, while also having rheological properties that allow them to spread easily over the skin. In one or more embodiments, the organogels are further characterized as being "substantially free" of surfactants having sufficient hydrophilicity to result in HLB values in excess of the requirements of the embodiments with regard to which such values are specified, i.e., "higher HLB surfactants". In one embodiment of this invention it is contemplated that the organogel is substantially free of surfactant having an HLB greater than 6 and, in the case of anhydrous organogels, is preferably substantially free of surfactant having an HLB greater than 4; in another embodiment of interest the organogel is substantially free of surfactant having an HLB greater than 5 and, in the case of anhydrous organogels, is preferably substantially free of surfactant having an HLB greater than 4. In yet another embodiment it is contemplated that the organogel is substantially free of surfactant having an HLB greater than that of the 12-hydroxystearic acid gelator of highest HLB value present in the organogel. In such context, "substantially free" means, based on the total weight of the organogel, an amount of less than 3% by weight, preferably less than 2% by weight, most preferably less than 1 % by weight of the referenced higher HLB surfactant, with organogels that are free of higher HLB surfactant being of particular interest in one or more embodiments.

HLB refers to the hydrophilic/lipophilic balance of a surfactant and may be calculated by the method known in the art as Griffin's method in which

$$HLB = 20^* Mh/M$$

where Mh is the molecular mass of the hydrophilic portion of the molecule and M is the molecular mass of the whole molecule. HLB values calculated in accordance with this method can range from 0 to 20 with an HLB value of 0 corresponding to a completely hydrophobic molecule and a value of 20 corresponding to a completely hydrophilic molecule.

Reducing or eliminating the amount of such higher HLB surfactants provides formulators with a means of producing milder, less irritating compositions. This is of particular interest in skin care and other leave-on applications. It is also of interest in conjunction with the use of less mild actives, for example, antiperspirant active such as aluminum chlorohydrate, where higher HLB materials may otherwise exacerbate the potential for astringency.

While one embodiment of the subject invention contemplates the use of organogel compositions that further include an astringent antiperspirant active, it has been found that the organogel itself can provide an axilla-blocking effect that achieves an antiperspirant benefit even in the absence of antiperspirant active. Thus, the organogels allow for the formulation of antiperspirants in which astringent antiperspirant active can be reduced or potentially eliminated.

Reduction of astringent antiperspirant active is of particular benefit in applications targeted for users that desire milder or, where astringent antiperspirant active is eliminated, more "natural" products.

In reference to the subject organogels, 12-hydroxystearic acid gelator refers to gelling agents selected from the group consisting of 12-hydroxystearic acid, fiber-forming esters of 12-hydroxystearic acid, fiber-forming salts of 12-hydroxystearic acid, and mixtures thereof. In one or more embodiments of particular interest the 12-hydroxystearic acid gelator is selected from 12-hydroxystearic acid and/or fiber-forming salts thereof, in particular the sodium and/or potassium salts of 12-hydroxystearic acid. In one or more embodiments the 12-hydroxystearic acid gelator is 12-hydroxystearic acid.

In addition to 12-HSA gelator, the composition may further comprise one or more co-structurants that do not detract from the ability of the 12-HSA gelator to form a network structure. Among the materials contemplated as co-structurants are additional small-molecule gelling agents. The term "small-molecule gelling agent" is herein understood to mean small organic molecules, including organic and inorganic salts thereof, capable of forming an organogel structure. In addition to 12-HSA, examples of small molecule gelling agents are, for example, alkane gelators such as, for example n-octacosane; ethers or thioethers, such as, for example crown ether $(CH_2CH_2O)_{20}$ or thioether $[H(CH_2)_{14}S(CH_2)_{14}H]$; substituted fatty acids other than 12-HSA; sorbitol derivatives; aliphatic amines; cholestanyl diocytadecyl amine, N-alkanamides, for example an alkanamide linked to a carbohydrate group, N-n-octyl-D-gluconamide; amino acid derivatives; peptides and urea derivatives.

As used herein the term "structurant(s)" refers to the 12-hydroxysteahc acid gelator, the crystal habit modifying copolymer, and co-structurant. Excluded from the term "structurant(s)" are viscosity and/or rheology modifying agents that do not impart a crystalline structure to the organogel, for example, ether-vinylic/anhydride maleic copolymers (e.g., polymer sold as "Viscofas"®); carboxyvinylic polymers such as those sold under the name Carbopol®; thixotropic agents; and the like. In one or more embodiments, structurant, i.e., 12-hydroxystearic acid gelator, crystal habit modifying copolymer and co-structurant, if present, account for from 4 to 30% by weight of the organogel. Amounts of preference depend, in part, on the stiffness of the gel desired. For stiffer gels it may be desirable to employ structurant in an amount of from 20 to 30% by weight, more particularly from 20 to 25% by weight, whereas, for less stiff gels amounts of structurant of from 4% to less than 20% by weight, more particularly from 10% to 15% by weight may be of interest. In one or more embodiments, 12-hydroxysteahc acid gelator comprises at least 60% by weight of the total structurant present in the organogel. In other embodiments the 12-hydroxysteahc acid gelator comprises at least 70% by weight of the total structurant, and in some embodiments, at least 80% by weight or even at least 90% weight of the total structurant present in the organogel. Also contemplated are compositions in which 12-hydroxysteahc acid gelator and crystal habit modifying copolymer are the sole structurants.

Without wishing to be bound by any theory of operation, it is believed that the vinyl amide copolymer employed herein functions as a crystal habit modifier. Desirably >70% by weight of the polymer, e.g., greater than 70 to 99% by weight, preferably >75 to 99% by weight of the polymer, comprises pendant alkyl groups and 1 to 30% by wt. of the polymer comprises a backbone monomer capable of bearing pendant groups. There may also be branches or

side chains on the backbone monomers that function like and count towards the >70% by weight of long chain alkyl groups. The pendant alkyl groups have chain length of $C_{12}$ to $C_{24}$, more particularly, $C_{14}$ to $C_{22}$ and even more particularly $C_{16}$ to $C_{20}$.

The pendant alkyl groups preferably comprise singly bonded linear hydrocarbon groups. The pendant alkyl groups should preferably have regularity. In the absence of such regularity (that is to say, the molecule is instead polydisperse), crystallization may not occur and desired gel structuring not form.

By regularity is meant that the pendant groups or branches (or side chains) attached to the main backbone chain(s) are, on average, of substantially the same length ($\pm$6 carbons, for example). This permits the chains to pack more readily and significantly enhance the crystallization process. In one or more embodiments, the pendant hydrocarbon groups or branches (or side chains) attached to the main backbone have the same length within $\pm$4 carbons, and preferably within $\pm$2 carbons.

As for the repeating backbone monomer, this is a vinyl amide monomer, preferably a cyclic vinyl amide polymer such as vinylpyrrolidone. Vinyl amide copolymers are preferably copolymers of vinylpyrrolidone and alpha-olefins. Of particular interest are copolymers having repeating units of the structure:

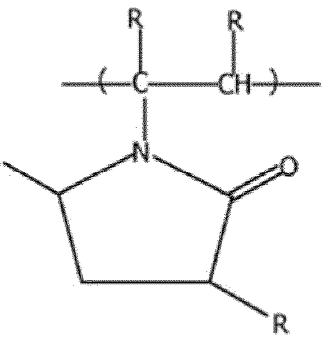

where R is independently H or alkyl group, with alkyl groups of particular interest having from 12 to 24, more particularly, from 14 to 22 carbon atoms. In at least one embodiment of particular interest R is independently hydrogen or 16 to 20 alkyl. In such repeating structures there are 4 positions possible for alkyl groups; in one embodiment of interest the majority of the long chain alkyl groups R will branch off the polyvinyl chain of the polymer backbone.

Such copolymers include, for example, copolymers of vinylpyrrolidone and alpha olefins commercially available, for example, from International Specialty Products under the designation Ganex®. The Ganex® copolymers have alkyl chains grafted directly onto a PVP chain such that the polymer backbone is more or less "coated" with "hairy" hydrocarbons, e.g., one monomer unit can carry up to four pendant hydrocarbon chains. Ganex® copolymers of particular interest include Ganex® V-216 (identified as a copolymer of N-vinyl-2-pyrrolidone and 1-hexadecene) and Ganex® V-220 identified as a copolymer of N-vinyl-2-pyrrolidone and 1-eicosene). Ganex® V-216 nominally comprises 80% of $C_{16}$ olefin and 20% of vinylpyrrolidone and is liquid at 25° and Ganex® V-220 nominally comprises 30% of vinylpyrrolidone and 70% of C20 olefin and is a low melting wax (melting point ~35°C).

Desirably, the crystalline habitat modifying copolymer nominally has a molecular weight of at least 5,000 daltons, preferably at least 7,000 daltons, e.g., 10,000-25,000 daltons to ensure gelation, however, depending upon the particular structure, copolymers of greater or lesser molecular weight may be employed herein. In at least one embodiment, the copolymer is desirably a liquid at 25°C.

The copolymer is desirably present in the organogel in an amount of from 0.05 to 3% by weight, preferably from 0.1 to 2% by weight. In one or more embodiments of particular interest the organogel is present in an amount of 0.2 to 1.5% by weight.

The subject organogels include one or more cosmetically acceptable oils. As used herein, the term "oil" refers to a water immiscible (alternatively described as hydrophobic or lipophilic) material that is liquid at a temperature of 20°C. Natural oils are of particular interest herein.

In one or more embodiments the natural oils desirably comprise one or more triglycerides of oleic acid, linoleic acid, linolenic acid and/or ricinoleic acid.

Various isomers of such acids often have common names, including linolelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include, for example, coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable natural oils are obtainable from hemp, which can be processed to derive stearadonic acid derivatives and maize corn oil. By virtue of its characteristics and availability soybean oil is of particular interest.

Yet other oils that may be employed herein include, for example, silicone oil, hydrocarbon oils, alcohol oils, ester oils, and ether oils. Such oils may be volatile or nonvolatile.

Silicone oils of interest are, for example, cyclomethicone, including for example cyclotetrasiloxane, cyclopentasiloxane and cyclohexasiloxane; polyalkylsiloxane, polyalkarylsiloxane and polyethersiloxane copolymers, including, for example, dimethicone and dimethicone copolyols, trimethylpentaphenyl trisiloxane, phenyltris(trimethylsiloxy)silane, tetraphenyl dimethyldisiloxane, and the like. Commercially available silicone oils are available from various suppliers including for, example, Dow Corning and Momentive.

Hydrocarbon oils suitable for use herein may be saturated or unsaturated. The hydrocarbon oils often contain from 12 to 40, more particularly, from 20 to 40 carbons on average and include mineral oils, hydrogenated polydecene, hydrogenated polyisobutene and the like.

Alcohol oils include, for example, branched chain monohydric alcohols containing from 12 to 40 carbon atoms, and often from 14 to 30 carbon atoms such as, for example, isostearyl alcohol.

Among the suitable ester oils are aliphatic esters, aromatic esters (which term as used in the instant specification and claims includes mixed aromatic/aliphatic ester oils), and triglyceride oils. Suitable aliphatic esters are esters that contain at least one long chain alkyl group such as esters derived from $C_1$ to $C_{20}$ alkanols esterified with a $C_6$ to $C_{22}$ alkanoic acid or $C_6$ to $C_{10}$ alkanedioic acid. Among the suitable aromatic esters are $C_8$-$C_{18}$ alkyl benzoates or mixtures thereof including, in particular, $C_{12}$-$C_{15}$ alkyl benzoates. Many suitable aromatic esters are available under the trademark Finsolv. Other aromatic esters which can be contemplated for use herein comprise double aromatic inclusion. Preferred double aromatic esters comprise a linear or branched alkyl chain, e.g. from 1 to 3 carbons, interposed between ester and/or ether substituted phenyl groups.

Ether oils suitable for use herein comprise liquid aliphatic ethers, including, for example, alkyl ethers of polypropylene glycol (PPG), the alkyl group comprising from 2 to 6carbon atoms and the PPG moiety comprising from 10 to 20 and particularly 14 to 18 propylene glycol units. One preferred ether oil bears the INCI name PPG14-butyl ether.

In at least one embodiment the cosmetically acceptable oil employed in the organogel comprises at least 50% by weight, preferably at least 75% by weight of natural oil. In embodiment of particular interest the cosmetically acceptable oil employed in the organogel comprises at least 90 percent by weight of natural oil, preferably triglyceride oil and most preferably soybean oil. In at least one embodiment of interest, exclusive of fragrance, the cosmetically acceptable oil employed in the organogel is comprised solely of natural oil.

Optionally a portion of the cosmetically acceptable oil may be replaced by water-immiscible materials that are semi-solid at 20°C. Semi-solids of interest herein include materials identified by CAS number 8009-03-8, known by the common names petrolatum, petroleum jelly, and soft paraffin.

The subject organogels can hold a relatively high level of oil. In one or more embodiments the ratio of the cosmetically acceptable oil to 12-hydroxystearic acid gelator is from 2:1 to 6.5:1, more particularly, from 3:1 to 6.0:1, even more particularly from 4:1 to 5.5:1. In one or more embodiments, the ratio of oil to total structurant is from 2:1 to 6:1, more particularly from 3.5.1: 5.5:1.

The organogels of interest may be "anhydrous" by which is meant that the amount of water present therein does not exceed 2% by weight of the organogel. In one or more embodiments the amount of water present in the organogel does not exceed 1% by weight and, even more particularly, is nominally 0%. In other embodiments, the organogels may include water and/or other hydrophilic components, for example, low molecular weight alcohols, such as, for example, $C_1$ to $C_4$ alcohols. In an organogel that is not anhydrous, the total amount of water typically will not exceed 30% by weight, with the amount of preference depending upon the end use application of interest. In one or more embodiments it is desirable that the total amount of water and lower, i.e., $C_1$-$C_4$, alcohols does not exceed 30% by weight of the organogel. The percentages given with respect to water are exclusive of water of hydration such as may be present in the astringent antiperspirant or other active ingredients.

Particularly when water or other hydrophilic components are present, it may be desirable for the organogel to further include one or more surfactants. Desirably such surfactants should not have HLB values in excess of 6 and preferably should not have HLB values in excess of 5. Among the surfactants contemplated for use herein are, for example, oleic acid, glycerol monolaurate, sorbitan monooleate, propylene glycol monolaurate, sorbitan monostearate and the like.

In another embodiment of the invention, the organogel may be formulated into aqueous or anhydrous cosmetic compositions (e.g., body or facial care compositions, antiperspirant composition, and the like) containing the structured, oil e.g., as part of the hydrophobic or fatty phase. Many such cosmetic preparations contain, at varying levels of concentration, a hydrophobic or fatty phase comprising a mixture of oil, a fat and/or wax. This is true, for example, for oil-in-water or water-in-oil emulsions, gels, oils for face and body care, milks and make-up products such as rouge or lipstick.

As a component of a cosmetic composition, the organogel may comprise 1 to 80%, for example of the total weight of the cosmetic composition, with higher levels of organogel being contemplated for some end use applications. In the case of many oil-in-water emulsions for body care compositions, the organogel will typically comprise 1 to 30%, preferably 2 to 15%, more preferably 2 to 10% by wt. of the cosmetic composition. Typically, water will comprise 70 to 99% by wt., more particularly from 80 to 90% of such compositions. Cosmetic compositions formulated as dispersions in other hydrophilic materials, e.g., low molecular weight alcohols and polyols, e.g. ethanol, propylene glycol, glycerol, and the like are also contemplated. In contrast to the organogel itself, which is desirably free of higher HLB surfactants, cosmetic compositions that include organogel as a component thereof may, depending upon the application for which they are intended, include one or more higher HLB surfactants, particularly when such compositions include hydrophilic materials.

The total amount of hydrophobic phase may vary and is typically from 1 to 99% by wt. of the cosmetic compositions depending upon the end use application. In many applications the organogel will comprise from 3 to 70% by weight, more particularly from 10 to 65% by weight of the cosmetic composition. As regards compositions having lesser amounts of hydrophilic components, the organogel will often reach levels of from 50 to 80% by weight of the cosmetic composition, with even higher levels of organogel being contemplated.

Other components which can be used in the hydrophobic or fatty phase of a cosmetic composition are vegetable,

animal or synthetic oils and/or wax. Suitable oils are as describe above with respect to the organogel. The waxes may be synthetic or naturally occurring or derived by processing of naturally occurring products, such as by hydrogenating unsaturated oils. Naturally occurring waxes or waxes derived from naturally occurring oils are often mixtures of compounds which include a substantial proportion, likely to be a majority, of fatty esters. Among waxes which may be used are castor wax, carnauba wax, jojoba wax, beeswax, ozokerite, candelilla wax, Montan wax and microcrystalline waxes.

As noted, cosmetic compositions may be all aqueous or anhydrous. The compositions may be fluid emulsions, lotions or more substantial emulsions. They may be, for example, milks or softening creams, milk or creams for hand and/or body care, makeup removing creams or milks, foundation bases, sunscreen milks or creams, artificial tanning milks or creams, milks or creams against perspiration, shaving creams or foams.

In one form, the cosmetic compositions may comprise primarily (>50%, preferably >55% by wt.) hydrophobic phase, of which 80%-100% of said hydrophobic phase is a structured oil, and which take the form of a sunscreen product, a hair care product, a body and/or skin care product, a pre-shave or after shave product, a bath oil, a gel, an ointment or a stick.

In one embodiment of particular interest, the organogel is provided in the form of or as part of an antiperspirant composition, with antiperspirant compositions in the form of creams or "soft solids" being of particular interest. Desirably such antiperspirant compositions are provided in packaging that includes instructions to apply same to the axilla and/or underarm and/or to apply same to achieve an antiperspirant benefit. As previously noted, such compositions may provide an antiperspirant benefit in the absence of antiperspirant active, however embodiments wherein antiperspirant active is present are also contemplated.

Antiperspirant active for use herein includes, in particular, aluminum, zirconium and mixed aluminum/zirconium salts, including both inorganic salts, salts with organic anions and complexes. Preferred astringent salts include aluminum, zirconium and aluminum/zirconium halides and halohydrate salts, such as chlorohydrates and activated aluminum chlorohydrates.

Aluminum halohydrates are usually defined by the general formula $Al_2(OH)_xQy \cdot wH_2O$ in which Q represents chlorine, bromine or iodine, x is variable from 2 to 5 and x+y=6 while $wH_2O$ represents a variable amount of hydration.

Zirconium actives can usually be represented by the empirical general formula: $ZrO(OH)_{2n-nz}B_z \cdot wH_2O$ in which z is a variable in the range of from 0.9 to 2.0 so that the value 2n-nz is zero or positive, n is the valency of B, and B is selected from the group consisting of chloride, other halide, sulfamate, sulfate and mixtures thereof. Possible hydration to a variable extent is represented by $wH_2O$. In one embodiment B represents chloride and the variable z lies in the range from 1.5 to 1.87. In practice, such zirconium salts are usually not employed by themselves, but as a component of a combined aluminum and zirconium-based antiperspirant.

The above aluminum and zirconium salts may have coordinated and/or bound water in various quantities and/or may be present as polymeric species, mixtures or complexes. In particular, zirconium hydroxy salts often represent a range of salts having various amounts of the hydroxy group. Aluminum zirconium chlorohydrate may be particularly preferred.

Antiperspirant complexes based on the above-mentioned astringent aluminum and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula $CH_2(NH_2)COOH$.

It is highly desirable to employ complexes of a combination of aluminum halohydrates and zirconium chlorohydrates together with amino acids such as glycine, examples of which are disclosed in U.S. Pat. No. 3,792,068 (Luedders et al). Certain of those Al/Zr complexes are commonly called AZG in the literature. AZG actives generally contain aluminum, zirconium and chloride with an Al/Zr ratio in a range from 2 to 10, especially 2 to 6, an Al/Cl ratio from 2.1 to 0.9 and a variable amount of glycine. Actives of this type are available from suppliers that include Summit Reheis. In one preferred embodiment the active is enhanced activity or activated aluminum/zirconium halohydrate, in particular, activated aluminum-zirconium tetrachlorohydrex glycine (AAZG).

Other actives which may be utilized include astringent titanium salts, for example those described in GB 2299506A.

In one or more embodiments it is desirable that the mean particle size of the antiperspirant salts is within the range of 0.1 to 100 micron with a mean particle size that is often from 3 to 30 microns, more particularly from 5 to 35 microns, and certain embodiments of interest from 10 to 25 microns. Actives having either larger or smaller mean particle sizes are also contemplated.

In embodiments where the organogel or an organogel-containing composition is formulated as an antiperspirant composition, the antiperspirant active may be present in the antiperspirant composition in an amount up to 30% by weight, more particularly from 10 to 30% by weight and commonly from 15 to 25% by weight, inclusive of water of hydration and any complexing agent that may also be present in the active. Use of the subject organogel may allow formulators to obtain desirable antiperspirant benefits empoying lower levels of astringent antiperspirant active, for example less than 15% by weight or in absence of such antiperspirant active entirely.

The antiperspirant composition can optionally comprise a supplementary deodorant active, i.e., an active other than the antiperspirant salt. Suitable supplementary deodorant actives can comprise deodorant effective concentrations of deoperfumes, and/or microbicides, including particularly bactericides, such as chlorinated aromatics, including biguanide derivatives, of which materials known as triclosan (Irgasan® DP300 from Ciba Specialty Chemicals), tricloban and chlorhexidine warrant specific mention. Supplementary deodorant actives are commonly employed at a concentration of from 0.1 to 5% by weight and often up to 1% by weight of the antiperspirant composition.

Other ingredients, conventional in the art of creams or soft solid antiperspirant compositions may be included in the antiperspirant compositions of the present invention. Optional ingredients include wash-off agents, often present in the antiperspirant compositions an amount of at least 0.05% by weight, and advantageously at least 0.25% by weight up to 5% by weight to assist in the removal of the composition from skin or clothing. When present, the wash-off agent is often present in an amount up to 1 %. Such wash-off agents are typically nonionic surfactants such as esters or ethers containing both a $C_8$ to $C_{22}$ alkyl moiety and a hydrophilic moiety which can comprise a polyoxyalkylene group (POE or POP) and/or a polyol, e.g., glycerol or sorbitol.

Non-limiting examples of other optional ingredients are drying agents, such as talc or aluminum starch octenylsuccinic, skin benefit agents such as allantoin, vitamins or lipids; colors; preservatives; skin cooling agents such as menthol and menthol derivatives; skin feel improvers such as high melting point polyethylene powder, micro-fine aluminum oxide powder and/or a particulate polymethylmethacrylate such as Ganzpearl® GMX-0810 from Ganz Chemical.

The amount of such optional adjuncts should not negatively impact the total solid content desired in the subject antiperspirants. When present, the total amount of such optional ingredients typically does not exceed 10% by weight of the composition and often does not exceed 5% by weight of the composition.

Fragrance is another common optional component in the antiperspirant and other cosmetic compositions contemplated by this invention. The total amount of fragrance (inclusive of all material present as part of fragrance encapsulate) is often from 0.001 to 5 wt.%, based on the total weight of the final composition, however higher levels may be of interest depending upon the intended end use.

In one embodiment, fragrance is desirably employed at a level of from 0.05 to 4 wt.%, more particularly from 0.1 to 3.5 wt%, based on the total weight of the final composition, be the composition an organogel or organogel-containing compositions.

The cosmetic compositions of this invention may include one or more rheology modifiers which add thixotropic body or aid in controlling syneresis. Such materials may also assist in processing of the composition while it is in molten form before being filled into containers. Non-limiting examples of such rheology modifiers include, for example, aluminum stearate, stearamide MEA, silica, in particular, finely divided silica such as fumed or precipitated silica, talc, and mixtures thereof. Silica is among the preferred rheological additives in one or more embodiments. When present, such rheology modifiers are desirably included in the cosmetic compositions in amounts, based on the total weight of the composition, of up to 8% by weight, with amounts of from 0.05 to 4.0% by weight, more particularly from 0.1 to 2% by weight being of interest in one or more embodiments.

In addition, the cosmetic composition may comprise various other components, typically at levels of 0.1-3% by wt. including coloring agents, perfumes, preserving agents, chelators, emulsifying agents, UV filters, pigments, pearlizing agents, pH adjusters, mineral or organic fillers and vitamins.

Method of Manufacture

The organogels according to the present invention can be made conveniently in accordance with processes that are typically employed to produce compositions containing 2-hydroxystearic acid gelators.

One suitable general method of manufacture comprises the steps of:

a) forming a mixture of the non-volatile oil and structurant;
b) heating the mixture to an elevated temperature at which the structurant is melted or dissolved in the oil phase;
c) cooling or allowing said resultant mixture to cool to temperature below the flash point of any volatile oil component;
d) introducing the volatile oil components, if any; and
e) cooling the mixture to temperature below the gelation temperature of the composition

The order of introduction of the other ingredients is at the discretion of the manufacturer. It will be recognised that optional ingredients, if any, can be introduced at a convenient step in the process. Thus, any temperature sensitive ingredient is desirably introduced into the composition shortly before the dispenser or pack that will contain same is charged, and preferably at a temperature within 10°C of the setting temperature.

EXAMPLES

As referred to herein, Large Amplitude Oscillatory Strain (LAOS) and Strain-Rate Frequency Superposition (SRFS) are the following test procedures, both of which are carried out using a rotational rheometer (plate-plate geometry, 25mm in diameter; gap of 1.5mm) at a temperature of 25°C.

Large Amplitude Oscillatory Strain (LAOS)

In this test, frequency is kept constant (1 rad/s) and strain amplitude ($\gamma_0$) increased from ~$10^{-2}$ to 100%. The values of G' (elastic modulus) and G" (elastic loss) are recorded as a function of strain amplitude. As reported by the test, "critical strain" refers to the value of strain corresponding to the deviation of oscillatory stress amplitude from linear dependence vs. strain amplitude, or the strain at which the elastic modulus becomes dependent on the strain amplitude.

Strain-Rate Frequency Superposition (SRFS)

This test follows the procedure described by Wyss, H.M et al. in "Strain-rate frequency superposition: A rheological probe of structural relaxation in soft materials." Physical Review Letters, 98 (2007), incorporated herein by reference. In this test, both frequency and the strain amplitude are changed simultaneously, but the product of strain amplitude and frequency, called strain rate amplitude, is constant. The test starts from large deformations (strain amplitudes) and low frequencies, so the gel is strongly deformed; at the end of the test, one probes the viscoelastic moduli of the compositions as the test occurs at low strain amplitudes.

Anhydrous and aqueous compositions according to the formulations described in Table 1 were prepared.

**Table 1**

| | **Comparative Example A** | **Example 1** | **Example 2** | **Comparative Example B** | **Example 3** |
|---|---|---|---|---|---|
| **Ingredient (% by wt.)** | | | | | |
| Ganex® V-220 | --- | | | | 0.6 |
| Ganex® V-216 | --- | 0.5 | 0.6 | --- | --- |
| 12-HSA | 18 | 18 | 18 | 15 | 15 |
| Soybean Oil | 82 | 81.5 | 81.4 | 85 | 84.4 |

**Table 1 Con't.**

| | **Example 4** | **Example 5** | **Comparative Example C** |
|---|---|---|---|
| **Ingredient (% by wt.)** | | | |
| Ganex® V-220 | --- | --- | --- |
| Ganex® V-216 | 0.3 | 0.5 | ---- |
| 12-HSA | 15 | 15 | 15 |
| Soybean Oil | 64.7 | 64.5 | 65 |
| Water | 20 | 20 | 20 |

Rheological measurements (elastic modulus (G') and loss modulus (G")) were obtained on the compositions of Example 2 and Comparative Example A using the large amplitude oscillatory strain (LAOS) and strain-rate frequency superposition (SRFS) tests described above.

LAOS test results for Example 2 and Comparative Example A are provided in Figure 1. The test shows that the Example 2 organogel with the Ganex® V-216 additive had a higher critical strain (i.e., could be deformed to a larger extent without fracturing) than the composition of Comparative Example A which lacked the Ganex® V-216 additive. The yield stress estimated from LAOS for Example 2 was ~1000 Pa compared to ~100 Pa for Comparative Example A.

SRFS test results for the Example 2 and Comparative Example A compositions are provided in Figure 2. The Example 2 organogel was shown to better resist high deformation than the composition of Comparative Example A as demonstrated by its G', G" crossover at a lower frequency. Additionally, the elastic modulus G' of the Example 2 composition was as found to be ~$10^5$ Pa at frequency as low as 0.2 rad/s, whereas for Comparative Example A the same value is reached only at ~8 rad/s. The data demonstrates that the Example 2 organogel is better able to withstand deformations and is less fragile than Comparative Example A.

SRFS testing was used to compare rheology properties of the Example 1 and 2 compositions. Data is reported in Figure 3. Comparing Examples 1 and 2, increasing the amount of Ganex V-216 led to a shift in the frequency at which G'/G" crossover occurred and lowered the viscoelastic modulus.

LAOS testing was also used to compare the elastic modulus G' of Example 4, Example 5, and Comparative

Example C in the linear viscoelastic regime (i.e. in the range where G' is independent from the strain amplitude $\gamma_0$). In such region, the elastic modulus of Example 4 was ~1.75 MPa and the elastic modulus of Example 5 was about ~1.10 Mpa. Comparative Example C was phase-separated so no soft solid was formed.

The difference in rheology profile of the Comparative Example A and Example 2 compositions was found to be consistent with the difference in physical appearance observed in the compositions. To the naked eye, Comparative Example A had an irregular appearance with visible oil separation; in contrast, Example 2 was uniform in appearance and showed no sign of oil separation.

The microscopic morphology of 12-HSA organogels with and without a crystal habit modifying copolymer was compared. Figure 4 are micrographs of the Example 3 and Comparative Example B compositions. As demonstrated by the micrographs, the Example 3 composition had more uniform structure in which the crystalline clusters more closely "match" the size of the inter-cluster boundaries than the Comparative Example B composition.

## Claims

1. An organogel comprising:

   (a) 4 to 20% by weight of 12-hydroxystearic acid gelator;
   (b) 45 to 95% by weight cosmetically acceptable oil;
   (c) 0.05 to 3% by weight of a vinyl amide copolymer that carries at least 70% by weight of the copolymer of pendant alkyl groups having chain length of $C_{12}$-$C_{24}$.

2. The organogel according to claim 1 that is substantially free of surfactant having an HLB value greater than 6.

3. The organogel according to claim 1 that is substantially free of surfactant having an HLB value greater than 5, with the proviso that the organgel is substantially free of surfactant having an HLB value greater than 4 if the organogel is anhydrous.

4. The organogel according to any preceding claim that is in the form of an antiperspirant product that is free of astringent antiperspirant.

5. The organogel according to any preceding claim wherein the cosmetically acceptable oil comprises natural oil comprising one or more triglycerides of oleic acid, linoleic acid, linolenic acid and/or ricinoleic acid vegetable oil.

6. The organogel according to any preceding claim wherein the cosmetically acceptable oil comprises soybean oil.

7. The organogel according to claim 6 wherein soybean oil comprises at least 75% by weight of the cosmetically acceptable oil present in the organogel.

8. The organogel according to any preceding claim that comprises up to 30% by weight of water.

9. The organogel according to any one of claims 1 to 7, wherein the organogel is anhydrous.

10. The organogel according to any preceding claim wherein the ratio of the cosmetically acceptable oil to the 12-hydroxystearic acid gelator is from 2:1 to 6.5:1.

11. The organogel according to any preceding claim wherein 12-hydroxystearic acid gelator comprises at least 80% by weight of total structurant present in the composition.

12. The organogel according to any preceding claim wherein the 12- hydroxystearic acid gelator is selected from the group consisting of 12- hydroxystearic acid, its fiber-forming salts and mixtures thereof.

13. The organogel according to any preceding claim wherein the vinyl amide copolymer is a copolymer of vinylpyrrolidone and alpha olefin that comprises repeat units of the formula:

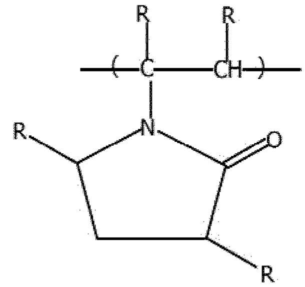

where R is independently hydrogen or $C_{16}$-$C_{20}$ alkyl.

**14.** The organogel according to any preceding claim that includes at least one additional co-structurant.

**15.** The organogel according to claim 9 wherein the 12-hydroxystearic acid gelator comprises at least 70% by weight of total structurant in the composition.

**16.** The organogel according to any preceding claim, wherein the organogel has crystalline domains having a domain size of on the order of from 1 to 100 microns.

**17.** A cosmetic composition that comprises an organogel as described in any preceding claim.

**Patentansprüche**

**1.** Organogel umfassend:

(a) 4 bis 20 Gew.-% 12-Hydroxystearinsäure-Geliermittel;
(b) 45 bis 95 Gew.-% kosmetisch akzeptables Öl;
(c) 0,05 bis 3 Gew.-% eines Vinylamid-Copolymers, welches mindestens 70 Gew.-% des Copolymers ausmachende seitenständige Alkylgruppen mit einer Kettenlänge von $C_{12}$ bis $C_{24}$ trägt.

**2.** Organogel gemäß Anspruch 1, welches im wesentlichen frei von Tensid mit einem HLB-Wert größer als 6 ist.

**3.** Organogel gemäß Anspruch 1, welches im wesentlichen frei von Tensid mit einem HLB-Wert größer als 5 ist, mit der Maßgabe, dass das Organogel im wesentlichen frei von Tensid mit einem HLB-Wert größer als 4 ist, sofern das Organogel wasserfrei ist.

**4.** Organogel gemäß irgendeinem vorhergehenden Anspruch, welches in der Form eines schweißhemmenden Produktes ist, das frei von einem adstringierenden Schweißhemmungsmittel ist.

**5.** Organogel gemäß irgendeinem vorhergehenden Anspruch, wobei das kosmetisch akzeptable Öl natürliches Öl umfasst, welches eines oder mehrere Triglyceride von Ölsäure, Linolsäure, Linolensäure und/oder Ricinolsäure-Pflanzenöl umfasst.

**6.** Organogel gemäß irgendeinem vorhergehenden Anspruch, wobei das kosmetisch akzeptable Öl Sojabohnenöl umfasst.

**7.** Organogel gemäß Anspruch 6, wobei Sojabohnenöl mindestens 75 Gew.-% des in dem Organogel vorhandenen kosmetisch akzeptablen Öls ausmacht.

**8.** Organogel gemäß irgendeinem vorhergehenden Anspruch, welches bis zu 30 Gew.-% Wasser umfasst.

**9.** Organogel gemäß irgendeinem der Ansprüche 1 bis 7, wobei das Organogel wasserfrei ist.

**10.** Organogel gemäß irgendeinem vorhergehenden Anspruch, wobei das Verhältnis des kosmetisch akzeptablen Öls

zu dem 12-Hydroxystearinsäure-Geliermittel 2:1 bis 6,5:1 beträgt.

**11.** Organogel gemäß irgendeinem vorhergehenden Anspruch, wobei das 12-Hydroxystearinsäure-Geliermittel mindestens 80 Gew.-% des gesamten in der Zusammensetzung vorhandenen Strukturbildners ausmacht.

**12.** Organogel gemäß irgendeinem vorhergehenden Anspruch, wobei das 12-Hydroxystearinsäure-Geliermittel aus der Gruppe bestehend aus 12-Hydroxystearinsäure, ihren faserbildenden Salzen und Mischungen davon ausgewählt ist.

**13.** Organogel gemäß irgendeinem vorhergehenden Anspruch, wobei das Vinylamid-Copolymer ein Copolymer von Vinylpyrrolidon und alpha-Olefin ist, welches Grundeinheiten der Formel

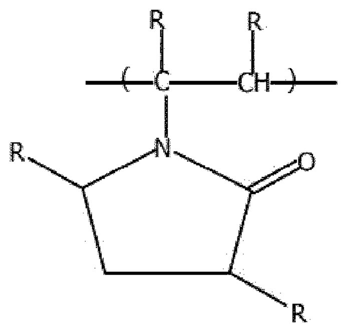

umfasst, wobei R unabhängig voneinander Wasserstoff oder $C_{16}$ bis $C_{20}$ Alkyl ist.

**14.** Organogel gemäß irgendeinem vorhergehenden Anspruch, welches mindestens einen zusätzlichen Co-Strukturbildner umfasst.

**15.** Organogel gemäß Anspruch 9, wobei das 12-Hydroxystearinsäure-Geliermittel mindestens 70 Gew.-% des gesamten Strukturbildners in der Zusammensetzung ausmacht.

**16.** Organogel gemäß irgendeinem vorhergehenden Anspruch, wobei das Organogel kristalline Domänen mit einer Domänengröße in der Größenordnung von 1 bis 100 Mikrometer hat.

**17.** Kosmetische Zusammensetzung, welche ein Organogel gemäß irgendeinem der vorhergehenden Ansprüche umfasst.

## Revendications

**1.** Organogel comprenant :

(a) de 4 à 20 % en poids d'un agent de formation de gel d'acide 12-hydroxystéarique ;
(b) de 45 à 95 % en poids d'une huile cosmétiquement acceptable ;
(c) de 0,05 à 3 % en poids d'un copolymère de vinylamide qui porte au moins 70 % en poids du copolymère de groupes alkyle suspendus présentant une longueur de chaîne de $C_{12}$-$C_{24}$.

**2.** Organogel selon la revendication 1, lequel est pratiquement exempt de tensioactif présentant une valeur HLB supérieure à 6.

**3.** Organogel selon la revendication 1 qui est pratiquement exempt de tensioactif présentant une valeur HLB supérieure à 5, à condition que l'organogel soit pratiquement exempt de tensioactif présentant une valeur HLB supérieure à 4 si l'organogel est anhydre.

**4.** Organogel selon l'une quelconque des revendications précédentes, qui est dans la forme d'un produit antiperspirant qui est exempt d'antiperspirant astringent.

**5.** Organogel selon l'une quelconque des revendications précédentes, dans lequel l'huile cosmétiquement acceptable comprend une huile naturelle comprenant un ou plusieurs triglycérides d'huile végétale d'acide oléique, d'acide linoléique, d'acide linolénique et/ou d'acide ricinoléique.

**6.** Organogel selon l'une quelconque des revendications précédentes, dans lequel l'huile cosmétiquement acceptable comprend de l'huile de soja.

**7.** Organogel selon la revendication 6, dans lequel l'huile de soja comprend au moins 75 % en poids de l'huile cosmétiquement acceptable présente dans l'organogel.

**8.** Organogel selon l'une quelconque des revendications précédentes qui comprend jusqu'à 30 % en poids d'eau.

**9.** Organogel selon l'une quelconque des revendications 1 à 7, dans lequel l'organogel est anhydre.

**10.** Organogel selon l'une quelconque des revendications précédentes, dans lequel le rapport de l'huile cosmétiquement acceptable à l'agent de formation de gel d'acide 12-hydroxystéarique est de 2:1 à 6,5:1.

**11.** Organogel selon l'une quelconque des revendications précédentes, dans lequel l'agent de formation de gel d'acide 12-hydroxystéarique comprend au moins 80 % en poids de structurant total présent dans la composition.

**12.** Organogel selon l'une quelconque des revendications précédentes, dans lequel l'agent de formation de gel d'acide 12-hydroxystéarique est choisi dans le groupe constitué d'acide 12-hydroxystéarique, ses sels formant des fibres et des mélanges de ceux-ci.

**13.** Organogel selon l'une quelconque des revendications précédentes, dans lequel le copolymère de vinylamide est un copolymère de vinylpyrrolidone et d'oléfine-alpha qui comprend des unités répétitives de la formule :

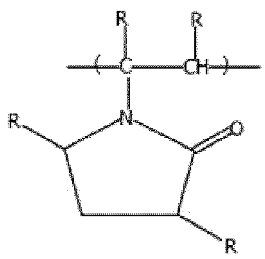

où R est indépendamment l'hydrogène ou un groupe alkyle en $C_{16}$-$C_{20}$.

**14.** Organogel selon l'une quelconque des revendications précédentes qui comprend au moins un co-structurant supplémentaire.

**15.** Organogel selon la revendication 9, dans lequel l'agent de formation de gel d'acide 12-hydroxystéarique comprend au moins 70 % en poids de structurant total dans la composition.

**16.** Organogel selon l'une quelconque des revendications précédentes, dans lequel l'organogel présente des domaines cristallins présentant une taille de domaine de l'ordre de 1 à 100 microns.

**17.** Composition cosmétique qui comprend un organogel selon l'une quelconque des revendications précédentes.

Fig. 1

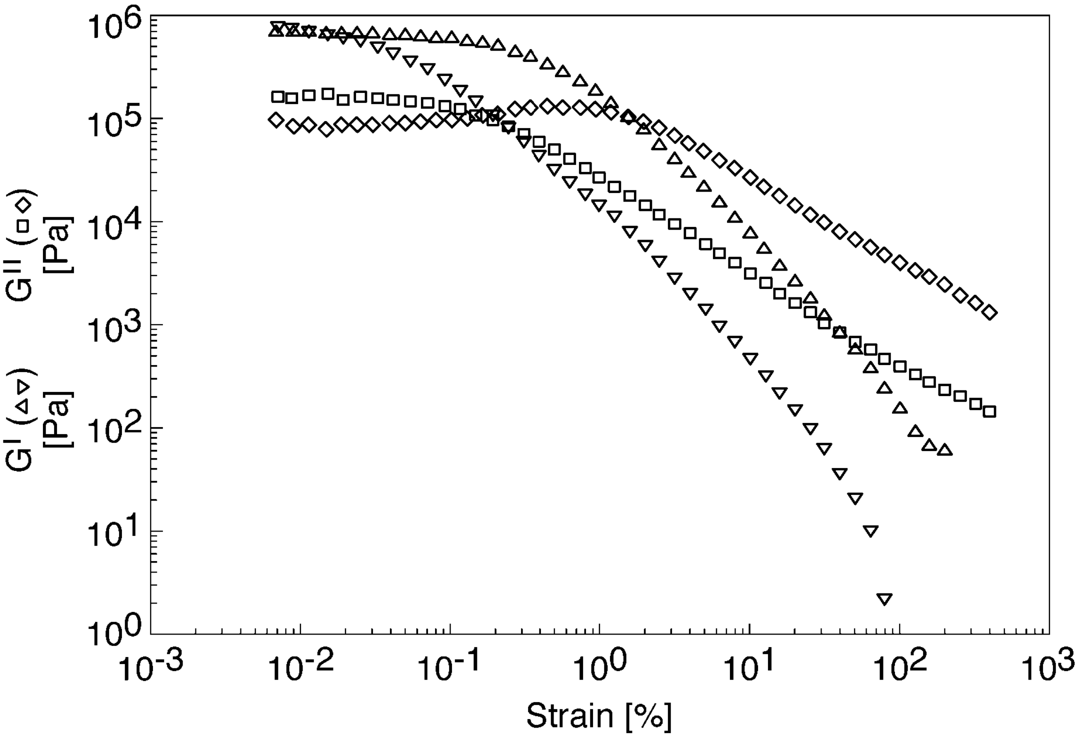
G'' (□◇) [Pa]
G' (△▽) [Pa]
10^6
10^5
10^4
10^3
10^2
10^1
10^0
10^-3
10^-2
10^-1
10^0
10^1
10^2
10^3
Strain [%]

Fig. 2

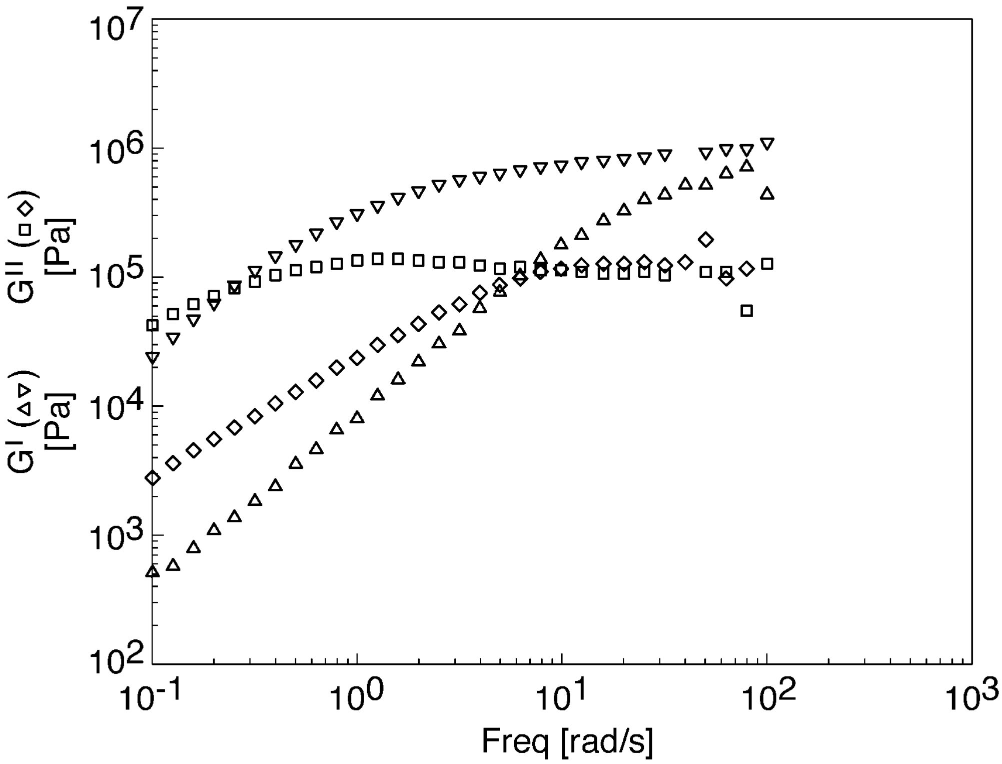
G'' (□◇) [Pa]
G' (△▽) [Pa]
10^7
10^6
10^5
10^4
10^3
10^2
10^-1
10^0
10^1
10^2
10^3
Freq [rad/s]

Fig. 3

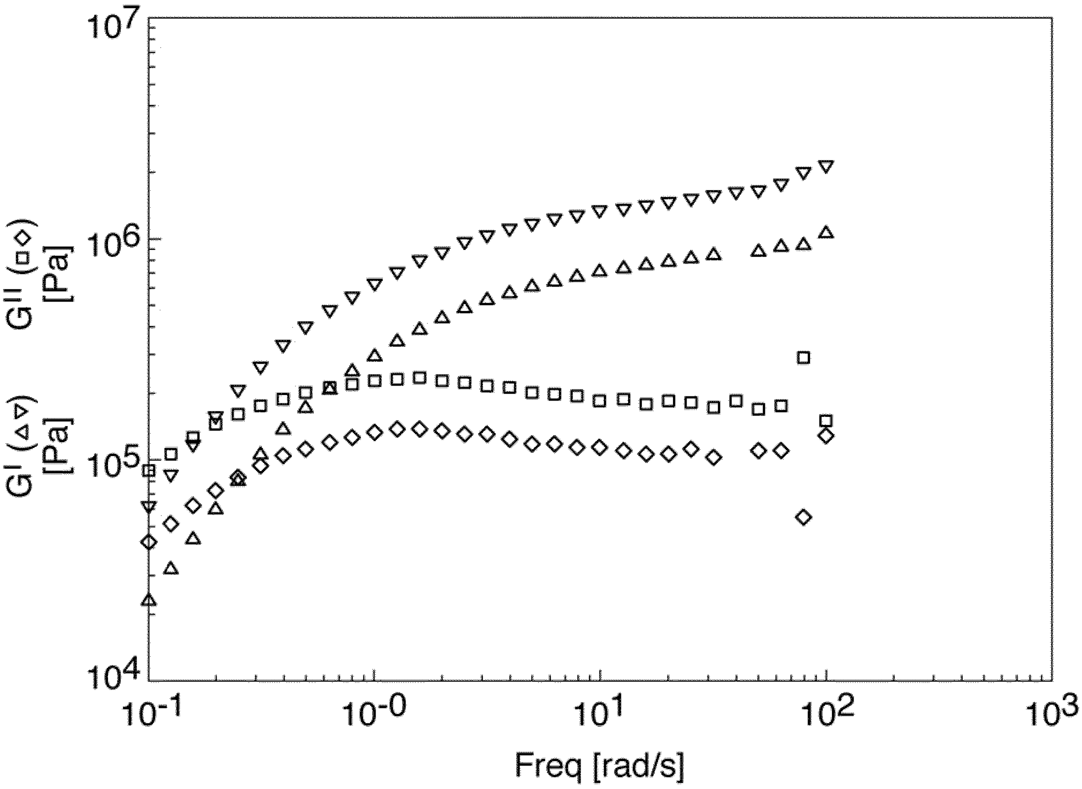


Fig. 4

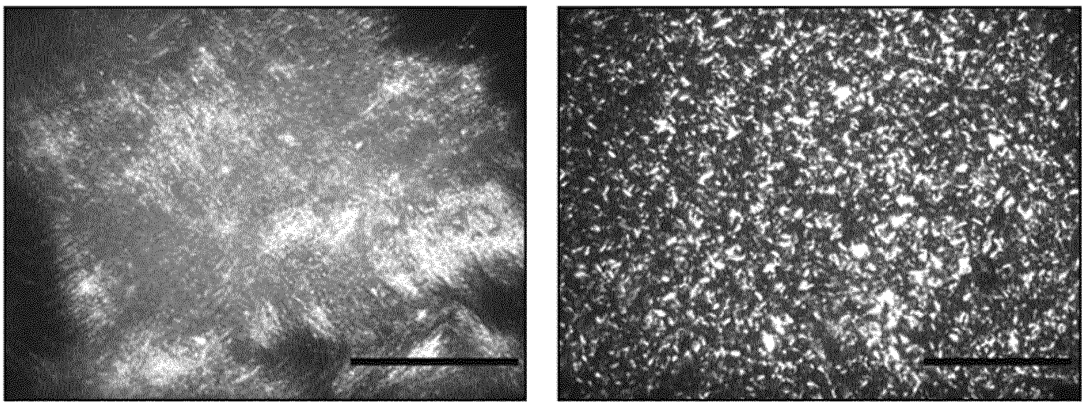

Scale 500μm

Scale 100μm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5429816 A
- US 6352688 B
- US 20090317341 A
- WO 9531961 A
- WO 0243685 A
- DE 10317751
- WO 2008037697 A
- WO 2010119035 A
- WO 2010119034 A
- US 3792068 A, Luedders
- GB 2299506 A


### Non-patent literature cited in the description


- **TSAU et al.** Thermoreversible Organogels of 12-Hydroxystearic Acid. *American Chemical Society Polymer Preprints,* 1994, vol. 35, 737-738
- **TAMURA et al.** Effect of Alkali Metal Ions on Gel Formation in the 12-Hydroxystearic Acid/Soybean Oil System. *JAOCX,* August 1991, vol. 68 (8
- **WYSS, H.M et al.** Strain-rate frequency superposition: A rheological probe of structural relaxation in soft materials. *Physical Review Letters,* 2007, 98